Europäisches Patentamt

European Patent Office

Office européen des brevets

(19)

(11) Numéro de publication : **0 140 725**
**B1**

(12) **FASCICULE DE BREVET EUROPÉEN**

(45) Date de publication du fascicule du brevet :
11.11.87

(51) Int. Cl.⁴ : **C 12 P 19/06, E 21 B 43/22**

(21) Numéro de dépôt : 84401663.4

(22) Date de dépôt : 13.08.84

(54) Procédé de traitement d'une solution de polysaccharide et son utilisation.

(30) Priorité : 30.08.83 FR 8313885

(43) Date de publication de la demande :
08.05.85 Bulletin 85/19

(45) Mention de la délivrance du brevet :
11.11.87 Bulletin 87/46

(84) Etats contractants désignés :
AT DE FR GB SE

(56) Documents cités :
EP-A- 0 039 962
EP-A- 0 049 012
GB-A- 2 099 008
CARBOHYDRATE RESEARCH, vol. 77, 1979, pages 289-292, Elsevier Scientific Publishing Co., Amsterdam, NL; G.J. WALKER et al.: "Hydrolysis of (1-3)-alpha-D-glucosidic linkages in oligosaccharides and polysaccharides by Cladosporium resinae exo-(1-3)-alpha-D-glucanase"
CHEMICAL ABSTRACTS, vol. 65, 1966, colonne 9252, point e, Columbus, Ohio, US; SHIRO HASEGAWA et al.: "Alpha-1,3-glucanase from a fungal source" & CHEM. IND. (London), 1966(25), 1033

(73) Titulaire : **RHONE-POULENC CHIMIE**
25, quai Paul Doumer
F-92408 Courbevoie Cédex (FR)

(72) Inventeur : **Gozard, Jean-Pierre**
**29, rue du Commandant Faurax**
**F-69006 Lyon (FR)**
Inventeur : **Jarry, Alain**
**Le Paradis - Maisonnais**
**F-79500 Melle (FR)**
Inventeur : **Lucioni, Alain**
**25, rue Docteur Faquin**
**F-69500 Bron (FR)**

(74) Mandataire : **Tavernier, Colette et al**
**RHONE-POULENC INTERSERVICES Service Brevets**
**Chimie 25, Quai Paul Doumer**
**F-92408 Courbevoie Cédex (FR)**

Jouve, 18, rue St-Denis, 75001 Paris, France

**0 140 725**

## Description

L'invention concerne un procédé de traitement d'une solution aqueuse d'hétéropolysaccharide, en particulier de gomme xanthane, en vue d'améliorer ses caractéristiques de filtrabilité et d'injectabilité. Le procédé est particulièrement adapté pour traiter des moûts entiers de fermentation destinés à la préparation de fluides aqueux pour le déplacement du pétrole des gisements partiellement épuisés.

Les hétéropolysaccharides ou biopolymères obtenus par fermentation d'un hydrate de carbone sous l'action de bactéries du genre Xanthomonas ou Arthrobacter ou de champignons appartenant au genre Sclérotium, ont trouvé de nombreuses applications industrielles en raison de leurs propriétés épaississantes ou viscosifiantes.

L'une des applications connues de ces hétéropolysaccharides de type Gomme Xanthane est la récupération secondaire et tertiaire du pétrole. Dans cette application, on utilise des solutions aqueuses diluées à une concentration d'environ 300 à 3 000 ppm pour déplacer l'huile des réservoirs partiellement épuisés. La Gomme Xanthane se caractérise en effet par une haute viscosité à basse concentration, une grande insensibilité à la salinité et à la nature des sels et une grande stabilité aux contraintes mécaniques. Toutefois les solutions préparées à partir des grades industriels soit à partir du moût de fermentation, soit par dilution de la poudre précipitée et séparée à partir du moût, présentent l'inconvénient majeur de colmater rapidement les pores des formations rocheuses dans lesquelles elles sont injectées, provoquant ainsi des augmentations de pression indésirables et empêchant rapidement toute récupération supplémentaire d'huile. On sait que les origines de ce colmatage sont dues à la présence d'une part de particules insolubles telles que débris cellulaires et bactéries non viables provenant de la fermentation, d'autre part d'un certain nombre d'agrégats translucides ou microgels notamment si la solution est préparée avec du biopolymère qui a été précipité à partir du moût de fermentation.

Différentes méthodes ont été proposées dans l'art antérieur pour améliorer la viscosité et/ou la filtrabilité et l'injectabilité des solutions de gomme xanthane, ces méthodes incluant des traitements thermiques, la floculation, des traitements enzymatiques, associés ou non à une filtration par exemple sur terres diatomées.

Des traitements thermiques ont été décrits notamment dans les brevets des Etats-Unis d'Amérique n° 3 555 447, n° 3 501 578, n° 3 729 460, n° 3 773 752, n° 3 801 502 ainsi que dans les demandes de brevet français publiées n° 2 330 697 et n° 2 440 992. Dans tous ces procédés, le traitement par la chaleur est réalisé soit au pH naturel de la solution aqueuse de polysaccharide, soit à un pH alcalin. L'utilisation de pH basiques provoque la désacétylation de la gomme xanthane et risque d'entraîner une dépolymérisation.

L'action des enzymes a aussi été proposée afin d'améliorer l'injectivité et la filtrabilité des solutions aqueuses de gomme xanthane.

Les brevets des Etats-Unis d'Amérique n° 3 966 618, n° 4 119 491, n° 4 165 257 décrivent des traitements à l'aide d'une enzyme de type protéase. Ces traitements ne permettent pas de surmonter complètement les problèmes de colmatage liés à la présence de matières non protéiniques insolubles.

Le brevet européen n° 0 039 962 décrit un traitement avec une enzyme complexe ayant une activité β 1,3-glucanase et protéase dérivée de Pellicularia filamentosa et/ou Pellicularia sasakii.

Finalement les demandes de brevet français publiées n° 2 491 494 et 2 506 328 proposent un traitement enzymatique par polysaccharase et par polysaccharase associé à protéase. Il a été constaté que le traitement par polysaccharase seul avait une efficacité limitée. Le traitement mixte polysaccharase et protéase est complexe à mettre en œuvre car les deux types d'enzyme développent leur activité dans des conditions de pH et de température différentes.

Il a été trouvé un traitement en une seule étape qui permet d'améliorer l'injectabilité et la filtrabilité des solutions diluées sans modification appréciable de leur pouvoir viscosifiant par rapport à des solutions qui n'ont pas été traitées.

Le procédé de traitement de solutions d'hétéropolysaccharides selon l'invention comprend un traitement à l'aide d'une enzyme complexe et est caractérisé en ce qu'il consiste à chauffer la solution aqueuse contenant 0,15 à 30 % en poids d'hétéropolysaccharide à un pH compris entre 3,5 et 6,2 à une température de 60 à 110 °C pendant 5 à 60 minutes et à contacter la solution avec une quantité efficace d'une enzyme complexe contenant de la mutanase à un pH de 3,5 à 7 pendant au moins 4 heures à une température inférieure à 60 °C.

Les mutanases constituent une famille d'enzymes dont le principal point commun est leur aptitude à attaquer les liaisons 1,3 glucosidiques en position alpha. Les mutanases sont produites par culture de microorganismes appropriés sur un milieu dont la source principale de carbone est le mutane provenant d'un Streptocoque, par exemple Streptococcus mutans CBS n° 350-71. Des microorganismes générateurs de mutanases comprennent Trichoderma harzianum, Penicillium funiculosum, Penicillium melinii et Penicillium janthinellum.

Le produit contenant de la mutanase que l'on utilise préférentiellement dans le procédé de l'invention est obtenu à partir de Trichoderma harzianum. La culture de Trichoderma harzianum avec pour objet la préparation d'une α 1,3 glucanase (mutanase) capable d'hydrolyser un α 1,3 glucane est décrite dans le brevet GB 1 373 487.

2

Dans les fermentations microbiennes enzymatiques, on produit, en l'absence de conditions particulières optimales, généralement plusieurs composants enzymatiques apparentés en proportions relativement constantes. Un produit industriel typique, qui est utilisé dans la pratique de l'invention, est vendu sous la marque commerciale Novozym 234 par Novo Industri A/S. Cette enzyme contient, à côté de l'activité principale mutanase, des activités cellulase, laminarinase, xylanase, chitinase et protéinase. Ces enzymes multiples contribuent aux résultats obtenus par le procédé selon l'invention.

Les hétéropolysaccharides utilisés dans le procédé de l'invention sont des colloïdes hydrophiles obtenus par fermentation d'un hydrate de carbone sous l'action de microorganismes. Ces microorganismes peuvent être par exemple des bactéries appartenan au genre xanthomonas et plus particulièrement les espèces décrites dans Bergey's Manual of Determinative Bacteriology (8e édition - 1974 - Williams N. Wilkins C° Baltimore) telles que xanthomonas begoniae, xanthomonas campestris, xanthomonas carotae, xanthomonas hederae, xanthomonas incanae, xanthomonas malvacearum, xanthomonas papaveri cola, xanthomonas phaseoli, xanthomonas pisi, xanthomonas vasculo rum, xanthomonas vesicatoria, xanthomonas vitians, xanthomonas pelargonii ; au genre arthrobacter et plus particulièrement les espèces arthrobacter stabilis, arthrobacter viscosus ; au genre erwinia ; au genre azotobacter et plus particulièrement à l'espèce azotobacter indicus ; au genre Agrobacterium et plus particulièrement les espèces Agrobacterium radiobacter, Agrobacterium rhizogenes, Agrobacterium tumefaciens ; ou des champignons appartenant au genre sclerotium et plus particulièrement aux espèces sclerotium glucanicum, sclerotium rolfii, etc.

L'expérience a montré que certaines espèces sont capables de produire les polysaccharides avec une efficacité particulière. L'espèce X. campestris convient tout particulièrement bien pour la synthèse de la Gomme Xanthane.

On peut faire fermenter toute une variété d'hydrates de carbone avec les microorganismes appartenant aux genres précités pour fabriquer l'hétéropolysaccharide utilisé dans le procédé selon l'invention. Les hydrates de carbone qui peuvent convenir comprennent le glucose, le saccharose, le fructose, le maltose, le lactose, l'amidon. La fermentation de l'hydrate de carbone se fait en général dans un milieu aqueux renfermant jusqu'à 100 g/l de glucide. On sait que le milieu de fermentation peut comprendre, en outre, une source de phosphore, une source de magnésium qui est un activateur d'enzymes, et une source d'azote qui peut être d'origine organique, minérale ou mixte organique minérale.

La préparation de la gomme Xanthane est décrite dans de nombreuses publications et de nombreux brevets. On peut se référer par exemple aux brevets US-A 3 020 206, US-A 3 020 207, US-A 3 391 060, US-A 4 154 654.

A l'issue du processus de fermentation et production de l'hétéropolysaccharide, le moût contient normalement environ 15 à 50 g/litre de polymère ainsi que divers constituants incluant des cellules bactériennes, des débris cellulaires, des protéines résiduelles et des ions minéraux.

Le biopolymère peut être récupéré du moût par précipitation à l'aide d'un agent de précipitation, par exemple l'isopropanol, filtration et séchage.

On peut appliquer le procédé de l'invention à des solutions obtenues par dissolution de biopolymère en poudre de qualité commerciale, mais dans une forme de réalisation avantageuse, et préférentielle pour emploi ultérieur en récupération du pétrole, on applique le procédé au bouillon entier de fermentation.

L'enzyme contenant de la mutanase est ajoutée à la solution aqueuse contenant de 0,15 à environ 30 % en poids de l'hétéropolysaccharide et le mélange est soumis à un vieillissement, avec ou sans agitation, à une température allant de la température ambiante à 60 °C, de préférence entre 25 et 55 °C, pendant une durée comprise entre environ 4 et 24 heures ou plus, et à un pH compris entre 3,5 et 7. Avantageusement, le pH est ajusté entre 4 et 5,8 par addition d'un acide minéral ou organique tel que l'acide sulfurique, l'acide phosphorique, l'acide nitrique, l'acide acétique, l'acide formique.

Quand la solution à traiter est préparée à partir de poudre isolée du moût de fermentation, la concentration en hétéropolysaccharide est avantageusement comprise entre 0,25 et 1,5 %.

Quand on traite un moût entier de fermentation, la concentration en hétéropolysaccharide est généralement comprise entre 1,5 et 20 % en poids. Le moût entier peut être lui-même le produit directement issu de la fermentation, auquel cas la concentration est normalement comprise entre 1,5 et 5 % ou bien il peut être un moût qui a été concentré par des méthodes classiques jusqu'à une teneur en hétéro-polysaccharide pouvant aller jusqu'à environ 20 %.

La quantité d'enzyme complexe que l'on ajoute à la solution aqueuse doit être suffisante pour assurer la dégradation des polysaccharides insolubles et des débris cellulaires bactériens.

Cette quantité dépend à la fois de l'activité enzymatique du complexe, de la teneur en matériaux insolubles de la solution à traiter et des conditions du traitement enzymatique.

En utilisant le complexe Novozym 234 une quantité comprise entre 0,1 et 3 % en poids par rapport au poids de l'hétéropolysaccharide est habituellement suffisante. La quantité utile peut encore être calculée par rapport à l'azote contenu dans le milieu provenant essentiellement de la biomasse. Des quantités comprises entre 20 et 150 g, de préférence entre 40 et 100 g d'enzyme pour 100 g d'azote sont en général suffisantes pour la destruction à la fois des résidus bactériens et des matériaux insolubles d'origine non bactérienne.

Le traitement enzymatique selon l'invention est également applicable aux solutions d'hétéropolysaccharides qui ont été préalablement clarifiées par des méthodes connues telles que pasteurisation, centrifugation, filtration sur terres diatomées...

La solution de l'hétéropolysaccharide, qui est avantageusement un moût entier de fermentation, est soumise avant ou après, mais de préférence avant traitement enzymatique à une opération de chauffage à une température comprise entre 60 et 110 °C pendant une durée de 5 minutes à 1 heure. Il a été trouvé que l'association du traitement thermique et du traitement enzymatique conduit à une clarification notablement supérieure des solutions de gomme Xanthane. Des résultats particulièrement intéressants au regard du pouvoir viscosifiant, de la filtrabilité et de l'injectabilité ont été obtenus par traitement thermique du moût de fermentation à un pH compris entre 3,5 et 6,2 de préférence entre 4 et 5,5 à une température de 60 à 110 °C et de préférence 80 à 100 °C pendant une durée de 5 à 60 minutes et de préférence 15 à 30 minutes, suivi d'un traitement par l'enzyme complexe.

Les solutions d'hétéropolysaccharides qui résultent du procédé de traitement enzymatique, de même que les poudres isolées à partir de ces solutions, sont particulièrement utiles dans toutes les applications nécessitant des produits clarifiés, par exemple dans les opérations de récupération secondaire et tertiaire du pétrole.

Une solution faiblement concentrée est un désavantage car elle ne peut être transportée économiquement. Le polymère sous forme de poudre pose des problèmes de redissolution sur le site d'utilisation. Il peut être avantageux dans certains cas de produire une solution concentrée de biopolymère. La concentration peut être réalisée par des moyens classiques comme l'évaporation ou l'ultrafiltration, cette dernière méthode étant préférée car plus économique et elle permet, de manière connue, et à l'échelle industrielle, de séparer les molécules de bas poids moléculaire des molécules de haut poids moléculaire et de concentrer les polymères sans modification de leurs propriétés rhéologiques. Il a été vérifié en particulier que l'ultrafiltration, même à gradient de vitesse élevé, n'amène pas de modification sur le pouvoir viscosifiant et la filtrabilité des solutions.

La concentration par ultrafiltration peut être réalisée en utilisant des techniques et équipements connus par exemple des appareils à plaques, à spirales, à tubes. On préfère les appareils à plaques rainurées dans lesquels le polymère peut être soumis à des gradients de vitesse élevés allant de 1 000 à 10 000 s$^{-1}$, abaissant la viscosité apparente du produit de manière sensible, ce qui permet un véhiculage rapide dans des appareils industriels de grande surface (10-50 m$^2$ unitaire) et un transfert amélioré. Des appareils de ce type sont décrits dans les brevets et demandes de brevets publiées FR-A 2 127 155, 2 141 417, 2 392 696, 2 400 380 et 2 471 507. On peut utiliser des membranes disponibles commercialement telles que des membranes cellulosiques, des membranes minérales, des membranes polymériques comme les membranes en polyamide, polybenzymidazole, copolymère acrylique, polyacrylonitrile, polysulfone, polyfluorure de vinylidène, polyélectrolytes complexes dont les seuils de coupure varient de 10 à 100 000.

Le flux est fonction de la température, de la pression, de la vitesse de passage ainsi que de la viscosité et de la concentration du biopolymère. Il est de l'ordre de 5 à 50 l/h · m$^2$ pour une vitesse linéaire de 0,5 à 5 m/s. On peut appliquer des températures allant de la température ambiante à environ 80 °C et des pressions de l'ordre de 1 à 15 bars, de préférence 1 à 6 bars.

L'ultrafiltration permet normalement d'obtenir des concentrations de 70 à 180 g de biopolymère par kg de moût. Si l'enzyme complexe est additionnée avant l'ultrafiltration, la réaction enzymatique se poursuit pendant la phase de concentration du polymère.

En variante, on peut encore purifier la solution de l'hétéropolysaccharide par diafiltration en lui ajoutant pendant ou après l'ultrafiltration de l'eau en continu, ou périodiquement, à une vitesse correspondant sensiblement à celle du prélèvement de l'ultrafiltrat. Les molécules de bas poids moléculaire, qui résultent de la réaction de dégradation enzymatique, sont éliminées dans le permeat. Le produit ainsi purifié présente, de manière inattendue, un pouvoir viscosifiant amélioré.

Le traitement selon l'invention peut être mis en œuvre en discontinu ou en continu. Il peut être intégré ou non à une unité de fermentation ou être effectué sur un site d'utilisation, tel qu'au voisinage d'un puits de pétrole. Dans un mode de mise en œuvre avantageux, le procédé est intégré à un traitement composite incluant traitement thermique en milieu acide, traitement enzymatique et concentration par ultrafiltration avec purification par lavage continu.

Il a été trouvé qu'un traitement thermique en milieu acide associé au traitement enzymatique permet d'améliorer les performances de l'ultrafiltration par rapport à un moût non traité thermiquement ou traité dans des conditions de pH neutre ou alcalin selon l'art antérieur. En particulier, pour une même perte de charge aux bornes de l'ultrafiltre, le traitement de chauffage en milieu acide permet d'obtenir des solutions de biopolymère plus concentrées pour des débits plus importants d'alimentation. On peut ainsi obtenir des concentrations allant jusqu'à 200 et même 300 g/kg de moût.

Les solutions d'hétéropolysaccharides qui résultent du procédé de traitement selon l'invention, de même que les poudres isolées à partir de ces solutions, sont utiles dans toutes les applications des gommes de type xanthane et plus spécialement dans les applications nécessitant des produits clarifiés et purifiés, par exemple dans le domaine alimentaire, pharmaceutique, et dans les opérations de récupération assistée du pétrole.

Les exemples suivants illustrent l'invention.

## Exemple 1

On utilise un moût de fermentation à 18 g/litre de gomme xanthane obtenu par fermentation avec Xanthomonas campestris.

Une fraction du moût est concentrée par ultrafiltration en utilisant un Module UFP 10 équipé de membranes IRIS 3038 (Marques commerciales de la Société Rhône-Poulenc Recherches) jusqu'à une concentration de 112 g de biopolymère par kg de moût.

Une seconde fraction est ajustée à pH 5,5 par addition d'acide sulfurique concentré puis est chauffée à 100 °C pendant 15 minutes. Après refroidissement le moût est concentré par ultrafiltration comme la première fraction jusqu'à une concentration de 112 g/kg.

A 1 kg de chaque fraction de moût concentré, on ajoute 1,1 g de Novozym 234 (Marque commerciale de Novo Industri A/S). Le mélange est maintenu 14 heures à 30 °C sous agitation puis est refroidi à température ambiante.

Sur le moût ainsi traité, on détermine la viscosité d'une solution à 1 000 ppm de gomme xanthane contenant 5 g/litre de NaCl, ainsi que la turbidité de la solution à 1 000 ppm.

Les mesures de viscosité sont effectuées à l'aide d'un viscosimètre Brookfield muni d'un adaptateur UL, à 25 °C, pour un taux de cisaillement de 7,3 s⁻¹.

La turbidité est déterminée par mesure de la densité optique à 650 nanomètre pour un chemin optique de 4 cm.

Les résultats figurent dans le tableau I.

### Tableau I

|  | Viscosité en mPa.s | Densité optique |
|---|---|---|
| Traité NOVOZYM 234 non traité thermique | 42,5 | 0,415 |
| Traité 100 °C/pH 5,5 puis traité NOVOZYM 234 | 63,1 | 0,144 |
| Traité 100 °C/pH 5,5 non traité NOVOZYM 234 (comparatif) | 59,5 | 0,749 |

## Exemple 2

On utilise un moût de fermentation contenant 80 g de gomme xanthane par kg de moût, qui a été concentré en continu par ultrafiltration à partir d'un moût brut à 15,6 g/kg, traité à pH 5,5 pendant 15 minutes à 100 °C et sans traitement thermique.

A 1 kg de moût concentré, on ajoute 1 g de Novozym 234. Le mélange est maintenu 14 heures à 40 °C sous agitation puis est refroidi à température ambiante.

L'efficacité du traitement sur l'aptitude à la filtrabilité et à l'injectabilité est déterminée selon les tests décrits ci-après, en comparaison avec un même moût n'ayant pas subi le traitement enzymatique.

Les résultats figurent dans le Tableau II.

Test d'écoulement ou de filtrabilité à débit constant

Ce test permet de mettre en évidence le phénomène de colmatage pouvant se produire lors de l'injection d'une solution diluée de biopolymère dans un gisement pétrolier, et donc de mesurer l'aptitude de la solution de biopolymère à être utilisée en R.A.P.

Le principe de ce test consiste à faire circuler les solutions diluées, à débit constant à travers un filtre calibré. La perte de charge (ΔP) engendrée aux bornes du filtre par le passage de la solution de biopolymère caractérise l'aptitude à la filtration.

Pour respecter les conditions d'utilisation sur champ, les solutions sont testées à viscosité identique (et non à concentration égale).

Les tests sont effectués dans les conditions suivantes :
— Température : 30 °C
— Débit : 22,5 ml/heure
— Filtres Millipore de 47 mm de diamètre, diamètre des pores 3 μm, 8 μm et 12 μm.
— Préparation des solutions. Le moût est dilué dans de l'eau saline (50 g/l NaCl et 5 g/l CaCl₂) de manière que la solution résultante ait une viscosité de 35 mPa·s (mesurée au viscosimètre Brookfield, adaptateur UL à 30 °C, taux de cisaillement 7,3 s⁻¹).
— La perte de charge ΔP est mesurée lorsque 350 ml de solution ont filtré. Si la pression excède 50 millibars, le nombre entre parenthèses indique le volume de la solution qui a filtré lorsque la pression atteint 50 mbar.

**0 140 725**

Test d'injection à pression constante

Ce test permet de mettre en évidence les propriétés d'injectabilité des solutions diluées de biopolymère. Le principe consiste à faire circuler les solutions, sous pression constante, à travers un filtre calibré. Le volume écoulé en fonction du temps caractérise l'injectabilité.

On opère dans les conditions suivantes :
— Filtres Millipore 47 mm de diamètre, diamètre des pores de 0,8 $\mu$m à 8 $\mu$m
— Pression 3 bars
— Préparation des solutions : identique au test de filtrabilité à débit constant
Viscosité 35 mPa·s (Brookfield - Adaptateur UL 30 °C — 7,3 s$^{-1}$)
— On note le temps écoulé pour le passage de 1 000 ml de solution. Si le temps excède 10 min, la valeur entre parenthèses indique le volume de la solution qui a filtré.

## Exemple 3

Cet exemple illustre l'influence du traitement enzymatique sur l'injectabilité des solutions diluées obtenues à partir de poudre de gomme xanthane qui a été précipitée du moût traité.

A partir du moût traité par le Novozym 234 de l'exemple 2, on isole le biopolymère par précipitation à l'isopropanol. Les fibres sont lavées, séchées et broyées.

A titre comparatif, le biopolymère est isolé à partir d'une fraction de même moût avant le traitement enzymatique.

5 g de poudre sont dilués dans de l'eau saline à l'aide d'un mélangeur Warnig Blender de telle sorte que la solution résultante présente une viscosité de 35 mPa·s et une concentration en sels de 50 g/l en Na Cl et 5 g/l en Ca Cl$_2$.

Chacune des solutions est soumise au test d'injection à pression constante décrit dans l'exemple précédent. Les résultats figurent dans le tableau III.

## Exemple 4

20 kg de moût fin de fermentation contenant 15,6 g de gomme xanthane par kg de moût sont traités thermiquement 15 min à 100 °C à pH 5,5.

Sur 5 kg de moût traité et refroidi à 40 °C, on ajoute 1 g de Novozym 234 et l'on maintient la solution 10 heures à 40 °C. Le moût est ensuite concentré par ultrafiltration sur un module UFP 2 (Marque commerciale de la Société Rhône-Poulenc Recherches) équipé de membranes IRIS 3038 A en acrylonitrile d'une surface filtrante de 0,022 m$^2$. Le moût est concentré jusqu'à une teneur de 80 g par kg de moût.

100 g de moût sont prélevés pour le test d'injectabilité à débit constant.

Le moût restant est lavé en continu (diafiltration) à 55 °C à l'eau permutée par 5 fois le poids du moût sur le même appareil et reconcentré à 80 g/kg. Au fur et à mesure du lavage et de la concentration finale la quantité d'azote dans le rétentat diminue pour atteindre en fin d'opération 20 % de la teneur initiale.

Les résultats du test d'injectabilité à débit constant figurent dans le Tableau IV en comparaison d'un même moût qui a été concentré par ultrafiltration sans traitement thermique et sans traitement enzymatique (témoin).

(Voir tableau II page 7)

6

Tableau II

| Traitement | | Filtrabilité à débit constant Δp en mbar | | | Injectabilité sous 3 bars Temps (en min) | | | | | Densité optique solutions à 1 000 ppm |
|---|---|---|---|---|---|---|---|---|---|---|
| 100 °C/15 min pH 5,5 | NOVOZYM 234 | 3 μm | 8 μm | 12 μm | 0,8 μm | 1,2 μm | 3 μm | 5 μm | 8 μm | |
| oui | néant | 50 (22,5 ml) | 44 | 6 | | | 10 min (260 ml) | 10 min (645 ml) | 1 min 0/ | 0,848 |
| oui | oui | 35 | 11,5 | 1,8 | 10 min (910 ml) | 9 min 18 | 1 min 21 | 32 sec | | 0,171 |
| néant | oui | 50 (83 ml) | 28,4 | 3,1 | | 10 min 210 ml | 10 min (450 ml) | 10 min (740 ml) | 48 sec | 0,390 |
| néant | néant | colmatage | | | | colmatage | | | | 0,860 |

Tableau III

| | Test d'injection à pression constante (3 bars) Temps pour passage de 1 000 ml de solution à 35 mPa.s sur filtres de priorité : (en micromètres) | | | |
|---|---|---|---|---|
| | 0,8 μm | 1,2 μm | 3 μm | 5 μm |
| Fibres d'un moût non traité | • | 10 min (220 ml) | 10 min (905 ml) | 30 sec |
| Fibres d'un moût traité au NOVOZYM 234 | 10 min (580 ml) | 1 min 52 sec | 32 sec | 19 sec |

Tableau IV

| Nature du moût | Solution testée | | Filtrabilité à débit constant Δp en m/bar | | |
|---|---|---|---|---|---|
| | Concentration (ppm) | Viscosité (mPa.s) | 3 μm | 8 μm | 12 μm |
| Concentré UF | 815 | 35 | 50 (22,5 ml) | 44 | 6 |
| Traité NOVOZYM 234 concentré UF | 815 | 35,3 | 37 | 12,1 | 1,7 |
| Traité NOVOZYM 234 concentré UF diafiltré | 750 | 37,2 | 35,1 | 11,4 | 1,8 |

**Revendications**

1. Procédé pour améliorer la filtrabilité des solutions aqueuses d'hétéropolysaccharides de fermentation comprenant un traitement à l'aide d'une enzyme complexe caractérisé en ce qu'il consiste à chauffer la solution aqueuse contenant 0,15 à 30 % en poids de l'hétéropolysaccharide à un pH compris entre 3,5 et 6,2 à une température de 60 à 110 °C pendant 5 à 60 minutes et à contacter la solution avec une quantité efficace d'une enzyme complexe contenant de la mutanase à un pH de 3,5 à 7 pendant au moins 4 heures à une température inférieure à 60 °C.

2. Procédé selon la revendication 1 caractérisé en ce que l'enzyme contient, à côté de l'activité mutanase, des activités cellulase, chitinase, protéinase, laminarinase et xylanase.

3. Procédé selon les revendications 1 ou 2 caractérisé en ce que l'hétéropolysaccharide est de la gomme xanthane.

4. Procédé selon l'une des revendications 1 à 3 caractérisé en ce que la solution aqueuse est un moût de fermentation.

5. Procédé selon la revendication 4 dans lequel le moût de fermentation a été concentré par ultrafiltration.

6. Procédé selon la revendication 1 caractérisé en ce que, avant ou après le contact avec l'enzyme, la solution est concentrée par ultrafiltration.

7. Procédé selon les revendications 5 ou 6 dans lequel l'ultrafiltration est réalisée sur un appareil à plaques rainurées à un gradient de vitesse de 1 000 à 10 000 s$^{-1}$.

8. Procédé selon l'une des revendications 1 à 7 caractérisé en ce que l'enzyme est ajoutée à la solution aqueuse en quantité comprise entre 0,1 et 3 % en poids par rapport au poids de l'hétéropolysaccharide.

9. Procédé selon l'une des revendications 1 à 7 caractérisé en ce que l'enzyme est ajouté à la solution aqueuse en quantité comprise entre 20 et 150 g pour 100 g d'azote provenant essentiellement de la biomasse.

10. Procédé selon l'une des revendications 1 à 9 caractérisé en ce que la solution traitée par l'enzyme contenant de la mutanase est finalement purifiée par diafiltration à travers une membrane poreuse.

11. Solutions aqueuses d'hétéropolysaccharides susceptibles d'être obtenues par un procédé selon l'une des revendications 1 à 10.

12. Emploi des solutions aqueuses selon la revendication 11 pour la préparation de solutions aqueuses diluées destinées à la récupération assistée du pétrole.

**Claims**

1. Process for improving the filterability of aqueous solutions of fermentation heteropolysaccharides, which comprises a treatment with an enzyme complex, characterized in that it consists in heating the aqueous solution containing 0.15 to 30 % by weight of the heteropolysaccharide at a pH of between 3.5 and 6.2 at a temperature of 60 to 110 °C for 5 to 60 minutes and in bringing the solution in contact with an effective amount of an enzyme complex containing mutanase at a pH of 3.5 to 7 for at least 4 hours at a temperature less than 60 °C. .

2. Process according to Claim 1, characterized in that the enzyme contains, besides mutanase activity, cellulase, chitinase, proteinase, laminarinase and xylanase activities.

3. Process according to Claims 1 or 2, characterized in that the heteropolysaccharide is xanthan gum.

4. Process according to one of Claims 1 to 3, characterized in that the aqueous solution is a fermentation must.

5. Process according to Claim 4, in which the fermentation must has been concentrated by ultrafiltration.

6. Process according to Claim 1, characterized in that before of after bringing into contact with the enzyme, the solution is concentrated by ultrafiltration.

7. Process according to Claims 5 or 6, in which the ultrafiltration is carried out in an apparatus with grooved plates at a velocity gradient of 1 000 to 10 000 $s^{-1}$.

8. Process according to one of Claims 1 to 7, characterized in that the enzyme is added to the aqueous solution in a quantity of between 0.1 and 3 % weight relative to the weight of the heteropolysaccharide.

9. Process according to one of Claims 1 to 7, characterized in that the enzyme is added to the aqueous solution in a quantity of between 20 and 150 g per 100 g of nitrogen originating essentially from the biomass.

10. Process according to one of Claims 1 to 9, characterized in that the solution treated with the enzyme containing mutanase is finally purified by diafiltration through a porous membrane.

11. Aqueous solutions of heteropolysaccharides capable of being obtained by a process according to one of Claims 1 to 10.

12. Use of the aqueous solutions according to Claim 11 for the preparation of dilute aqueous solutions intended for use in enhanced oil recovery.

**Patentansprüche**

1. Verfahren zur Verbesserung der Filtrierbarkeit von wäßrigen Lösungen von Heteropolysacchariden aus der Fermentation umfassend eine Behandlung mit Hilfe eines komplexen Enzyms, dadurch gekennzeichnet, daß man die wäßrige Lösung, die 0,15 bis 30 Gew.-% Heteropolysaccharid enthält bei einem pH von 3,5 bis 6,2 während 5 bis 60 Minuten auf eine Temperatur von 60 bis 110 °C erhitzt und die Lösung während mindestens 4 Stunden bei einer Temperatur unterhalb 60 °C bei einem pH-Wert von 3,5 bis 7 mit einer wirksamen Menge eines komplexen Enzyms, das die Mutanase enthält, in Berührung bringt.

2. Verfahren nach Anspruch 1, dadurch gekennzeichnet, daß das Enzym zusätzlich zur Mutanase-Aktivität Cellulase-, Chitinase-, Proteinase-, Laminarinase- und Xylanase-Aktivitäten enthält.

3. Verfahren nach Anspruch 1 oder 2, dadurch gekennzeichnet, daß das Heteropolysaccharid Xanthangummi ist.

4. Verfahren nach einem der Ansprüche 1 bis 3, dadurch gekennzeichnet, daß die wäßrige Lösung eine (konzentrierte) Fermentationsbrühe ist.

5. Verfahren nach Anspruch 4, dadurch gekennzeichnet, daß die Fermentationsbrühe durch Ultrafiltration eingeengt worden ist.

6. Verfahren nach Anspruch 1, dadurch gekennzeichnet, daß die Lösung vor oder nach dem in Berührung bringen mit dem Enzym durch Ultrafiltration eingeengt worden ist.

7. Verfahren nach den Ansprüchen 5 oder 6, dadurch gekennzeichnet, daß die Ultrafiltration in einem Apparat mit gerillten Platten mit einem Geschwindigkeitsgradienten von 1 000 bis 10 000 $s^{-1}$ durchgeführt wird.

8. Verfahren nach einem der Ansprüche 1 bis 7, dadurch gekennzeichnet, daß das Enzym in einer Menge von 0,1 bis 3 Gew.-%, bezogen auf das Gewicht des Heteropolysaccharids, zur wäßrigen Lösung gegeben wird.

9. Verfahren nach einem der Ansprüche 1 bis 7, dadurch gekennzeichnet, daß das Enzym in einer Menge von 20 bis 150 g je 100 g Stickstoff, im wesentlichen aus der Biomasse, zur wäßrigen Lösung zugesetzt wird.

10. Verfahren nach einem der Ansprüche 1 bis 9, dadurch gekennzeichnet, daß die mit dem Enzym behandelte Lösung, die die Mutanase enthält, abschliessend durch Diafiltration durch eine poröse Membran gereinigt wird.

11. Wäßrige Lösungen von Heteropolysacchariden, die durch ein Verfahren nach einem der Ansprüche 1 bis 10 erhalten werden können.

12. Verwendung der wäßrigen Lösungen nach Anspruch 11 für die Herstellung von verdünnten wäßrigen Lösungen, die für die sekundäre und/oder tertiäre Ausbeutung von Erdöllagerstätten bestimmt sind.